# EUROPEAN PATENT SPECIFICATION

(11) **EP 2 227 192 B1**
(45) Date of publication and mention of the grant of the patent: **21.09.2011**
(21) Application number: 08856311.9
(22) Date of filing: 28.11.2008
(51) Int. Cl.: A61F 2/90, A61F 2/88, A61F 2/06, A61F 2/84, A61F 2/82, A61F 2/00

(54) **IMPLANTABLE VESSEL SUPPORT**
IMPLANTIERBARE GEFÄSSSTÜTZE
SUPPORT DE VAISSEAU IMPLANTABLE

(30) Priority: 06.12.2007 DE 102007060497
(43) Date of publication of application: 15.09.2010
(60) Divisional application: 11168874.3
(73) Proprietor: Translumina GmbH, 72379 Hechingen (DE); Joline GmbH & Co. KG, 72379 Hechingen (DE)
(72) Inventor: KAISER, Siegfried, 72108 Rottenburg (DE); GERINGER-FITIS, Galina, 72414 Rangendingen (DE); EISENLOHR, Michael, 72379 Hechingen (DE); LEHMANN, Michael, 72070 Tübingen (DE); BEHNISCH, Boris, 72072 Tübingen (DE); KUNZ, Steffen, 72379 Hechingen (DE)
(74) Representative: Witte, Weller & Partner
(86) International application number: PCT/EP2008/010102
(87) International publication number: WO 2009/071243

(56) References cited:
- EP-A- 1 512 381
- US-A1- 2005 177 221
- US-A1- 2006 025 849
- US-A1- 2007 016 280
- US-A1- 2007 276 460

## Description

The present invention relates to a radially expandable vessel support for implantation in a secondary vessel branching off from a main vessel at a vessel opening and which, in the expanded state, bears on the inside wall of the secondary vessel.

A vessel support of this kind is known from US 2006/0025849 A1 US 2007/0276460 A1 and EP 1512381 A2.

In the context of the present invention, secondary vessel and main vessel are to be understood as meaning not only the branching of a smaller vessel from a larger vessel, but also the branching or dividing of vessels in general, in which case the main vessel and the secondary vessel can have approximately identical or also different diameters.

Vessel supports within the meaning of the present invention are intravascular implants, also designated as stents. Such stents are radially expendable endoprostheses that are implanted transluminally into blood vessels, the oesophagus, the trachea, the intestinal canal, etc., and are then radially expanded.

Stents are used, for example, to strengthen blood vessels and/or to prevent restenosis in the vessel system following angioplasty. They can be self-expanding or are actively expanded by a radial force applied from the inside, for example if they are fitted on a balloon.

Therefore, "radially expandable vessel support" within the context of the present invention is to be understood as meaning both self-expanding and also actively expandable vessel supports.

The vessel supports thereby have a hollow cylindrical body with a wall made up of interconnected struts and branches that form a radially permeable and resilient structure. In an often preferred design, the hollow cylindrical body comprises annular segments made up of zigzag-shaped and closed branches that form loops which lie alongside one another in the circumferential direction and which have proximal and distal tips, such that each segment has a meandering, undulating structure in the circumferential direction. The individual segments are interconnected by short struts. Upon expansion, the distance between the proximal and distal tips shortens slightly in each segment, but the zigzag structure is retained.

In the expanded state, the external diameter of the vessel support body corresponds approximately to the internal diameter of the vessel which is to be supported and on whose wall the vessel support bears, exerting a radial force that results from its flexible and resilient structure. In the longitudinal direction, the body of the vessel support is open in order to permit the passage of media or substances transported through the supported vessel.

In stents in particular, it is also known to coat their surfaces with medicaments, for example rapamycin, or to provide medicament reservoirs in the structure of the stents, or to use microporous struts and branches for intermediate storage of the medicament. The medicament is then delivered locally to the vessel wall, for example in order to avoid restenosis caused by proliferation of the surrounding tissue. Thus, by means of suitably coated stents, or stents provided with reservoirs, it is possible for medicaments to be delivered to the surrounding tissue as it were *in situ*. The coating of stents, i.e. of vascular prostheses, with medicaments is also desirable because this improves the biocompatibility of the implants, as a result of which it is possible, for example, to avoid the formation of thromboses on surfaces coming into contact with blood.

Stents that can be provided with a coating of various active substances have been described numerously in the prior art; see, for example, DE 202 00 220 U1, EP 0 875 218 A2 or EP 0 950 386 A2. These stents are generally placed in the body with the aid of so-called insertion systems and are released at the site of use, for which purpose they are loaded on catheters which are advanced through the corresponding vessels by the so-called Seldinger technique, using guide wires that run through the inner lumen of the catheter. At the intended site of use, the position of the stent is first checked, for example by means of X-ray markers mounted on the stent, and it is readjusted, if appropriate. Thereafter, the stent is expanded either by inflation of the balloon onto which the stent has been crimped, or by withdrawal of the protective sleeve with which a self-expanding stent is compressed during insertion.

Particular problems arise when vessels have to be stented in the area of ramifications or bifurcations. For this purpose, stents are generally implanted both into the main vessel and also into the secondary vessel, particular care being taken to ensure that the stent in the main vessel does not occlude the opening from the main to the secondary vessel.

To do this, in a method known from US 2004/0186560 A1, a main stent is first inserted into the main vessel, and a secondary stent is then inserted into the secondary vessel through the branches and struts of the main stent. Conversely, it is known from DE 10 2006 009 996 A1 to first insert the secondary stent such that part of this stent protrudes into the main vessel, and then to insert the main stent by means of which, upon expansion, that part of the secondary stent protruding into the main vessel is pressed against the wall of the main vessel around the vessel opening.

A disadvantage of these systems is that the positioning of the main stent is difficult and complicated, since its lateral opening must be aligned exactly with the secondary vessel.

In some designs, depending on the angle at which the secondary vessel branches off from the main vessel, the secondary vessel also has areas of variable size which lie opposite the vessel opening and on which the vessel support does not bear, such that the supporting function is not ensured in these areas, nor is the supply of medicaments. Moreover, there is considerable overlapping of parts of both stents in the main vessel with, in the case of blood vessels, the resulting accumulation of material contributing to the formation of clots and/or causing vortices that impede the transport of blood.

In order to solve these problems, the aforementioned US 2004/0186560 proposes that the secondary stent be beveled according to the angle between the two vessels, such that the unprotected area of the secondary vessel is minimized or completely avoided.

However, problems also arise here. On the one hand, the angle varies depending on the vessel and the patient, such that the bevel of the secondary stent has to be selected on an individual basis, which means that it is necessary to keep a large supply of stents with different diameters and bevels. On the other hand, the positioning of the secondary stent is also difficult and complicated here, whereby further a secure fit of the secondary stent is not always guaranteed.

In order to anchor the secondary stent securely on the vessel opening, it is known from US -2005/177221 A1 to anchor the secondary stent in the secondary vessel and to provide on the secondary stent an end section that protrudes into the main vessel. The end section is made up of structures which are distributed about the circumference and separate from one another about the circumference and which extend axially and are widened in a funnel shape and in this way are placed around the vessel opening, such that they bear on the inside wall of the main vessel around the vessel opening. The main stent then fixes this end section against the wall of the main stent surrounding the vessel opening.

There is a danger here of the secondary stent being compressed upon expansion of the main stent or of being completely pushed back into the secondary vessel. Moreover, there is an accumulation of material in the overlap area of the two stents, which is undesirable as it constitutes a starting point for restenosis and formation of clots.

The vessel support known from US 2006/0025849 A1, mentioned at the outset, is a secondary stent in which an end section is designed as a separate stent segment which is set apart from the secondary stent and has branches that in the circumferential direction extend in a zigzag shape, and which separate stent segment is connected to the distal main section via connectors that in the axial direction extend in an undulating or meandering shape.

The connectors are of such a length that the separate stent segment comes to lie in the main vessel alongside the vessel opening, while the secondary stent is anchored in the usual way in the secondary vessel. By suitable balloon dilation, the separate stent segment and the connectors in the main vessel are expanded to such an extent that the main stent can be pushed through them. Upon expansion of the main stent, the latter presses the separate stent segment and the connectors further apart and fixes them internally about the full circumference of the wall of the main vessel. The separate stent segment comes to lie completely in the main vessel and is adapted to the internal diameter of the main vessel.

On the one hand, the disadvantage already discussed arises here, namely that the main stent and the separate stent segment of the secondary stent overlap each other in the main vessel, which leads to an undesirable accumulation of material. On the other hand, the secondary stent itself does not completely cover the secondary vessel in the area of the wall lying opposite the vessel opening, whereby the connectors extending in the axial direction in this uncovered area are neither able to ensure the necessary mechanical support nor provide a sufficient supply of medicaments.

A further disadvantage is that, in the state when crimped onto the balloon, the relatively long connectors protrude radially outwards or, upon bending of the catheter, lift away from the balloon, such that they can catch in the vessel during the advance of the catheter.

In view of the above, the object of the present invention is to improve the known vessel support in such a way that the abovementioned disadvantages are avoided.

According to the invention, this object is achieved by the vessel support of claim 1.

The object underlying the invention is completely achieved in this way.

The inventors of the present application have in fact recognised that it is possible to design the vessel support with two sections that are arranged directly one behind the other in the axial direction and that may be interconnected via one or more short struts, where the main section anchors itself in the secondary vessel, and the proximal segment is designed such that it comes to lie in the vessel opening when the vessel support is expanded. The proximal segment can be expanded to such an extent that it comes into contact both with the wall of the secondary vessel and also with the wall of the main vessel.

In other words, the expanded proximal segment extends simultaneously over both vessels. It thus lies with one part of its circumference on the wall of the secondary vessel and with the rest of its circumference on the wall of the main vessel, specifically where these walls each lie opposite the vessel opening. The proximal segment extends as it were partially into the main vessel. A further vessel support can be introduced into the main vessel through this proximal segment and, upon expansion, fixes the proximal segment against the wall of the main vessel.

In this way, the wall of the secondary vessel is mechanically supported by the vessel support even in its area lying opposite the vessel opening, and medicaments can also be made available locally there by means of suitable design of the proximal segment.

Furthermore, there is only a slight overlap between the two vessel supports, since the proximal segment only lies with part of its circumference in the main vessel.

When the proximal segment is widened partially into the main vessel during expansion, the stent material of the proximal segment distributes itself over a greater circumference such that, compared with known secondary vessel supports, less stent material bears on the vessel walls, which likewise contributes to less overlapping between main stent and secondary stent.

A further advantage is that the novel vessel support can be crimped easily and securely onto a balloon, since there are no long connectors that extend between the proximal segment and the main section and that can bend upwards from the balloon.

The invention further relates to a radially expandable main vessel support for implantation in a section of a main vessel from which a secondary vessel branches off at a vessel opening, wherein the main vessel support comprises an approximately central area which lies opposite the vessel opening after expansion and is then substantially free of vessel support material, this main vessel support used together with the novel vessel support for the secondary vessel.

An advantage here is that the main vessel support is designed such that, upon expansion, an aperture is formed in the vessel support, and, with suitable positioning, this aperture lies opposite the vessel opening. This ensures that the vessel support positioned in the main vessel does not even partially occlude the vessel opening, such that the transport of blood or other media is not impeded by struts or branches of the main vessel support lying in front of the vessel opening.

In the novel secondary vessel support, it is preferable if the proximal segment is connected to the main section via at least one strut, the proximal segment preferably comprising a closed ring structure and also preferably extending in a spiral-shape from the main section and comprising zigzag-shaped or wave-shaped branches and/or struts.

It is of advantage here that, for the individual segments of the vessel support, a tried and tested structure is chosen which guarantees good mechanical support and supply with medicaments and also ensures a small overlap of the vessel support material of the secondary vessel support with that of the main vessel support.

It is particularly preferable if the main section is made up of a plurality of segments that are arranged one behind another in the axial direction and that are connected to one another via axially extending struts, whereby it is preferred if the proximal segment comprises loops which, in the axial direction, have a greater length than the segments of the main section and/or if the proximal segment comprises a greater number of loops than the segments of the main section.

These measures are of advantage structurally since they ensure that the proximal segment, by virtue of the material stored in its greater axial length and/or greater number of loops, can easily extend into the main vessels.

It is also preferable if the vessel support is fixed on a balloon of a balloon catheter, in which case the balloon, at its proximal end, preferably permits a greater expansion than does the rest of the balloon and is preferably sphere-shaped there.

The advantage here is that, through this design of the balloon, the secondary vessel support can be well and safely expanded, and the proximal segment reliably assumes its intended position in both vessels.

Further, in the main vessel support, it is preferred if at least the approximately central area is designed as a spiral-shaped circumferentially extending branch which preferably comprises axially extending struts or, alternatively, comprises a connection gap.

This spiral-shaped configuration has the advantage that no positioning problem arises, since the spiral-shaped area can be deformed in the area of the vessel opening, largely independently of its axial or circumferential position; such that it completely uncovers the vessel opening.

It is also of advantage that the spiral-shaped area covers the wall of the main vessel lying opposite the vessel opening in such a way that spiral-shaped gaps as it were remain, which has the effect that the overlap with the proximal segment of the secondary vessel support is reduced still further.

Of course, the entire vessel support can also be designed with a continuous spiral-shaped branch which, if appropriate, is stabilized by axial struts, whereby the spiral-shaped or coiled branch extends between a proximal segment and a distal segment, each segment being a closed ring.

It is preferable if the main vessel support is fixed on a balloon catheter whose balloon comprises, between its distal and proximal sections, an approximately central section that permits a greater expansion than the proximal and distal sections, in which case the balloon is preferably approximately spherical in the central section.

The advantage here is that the balloon with the central sphere reliably presses the branches of the central area out of the area of the vessel opening.

It is generally preferable if the vessel supports are provided with at least one position marker, preferably an X-ray marker, which can be mounted on the vessel support itself and/or on the balloon and/or catheter of the balloon catheter, so as to be able to monitor the positioning of the vessel support.

The novel secondary vessel support is preferably provided with the at least one position marker in the area of the proximal segment, in which case the position marker is preferably arranged in the area of the proximal end of a balloon of a balloon catheter:

The main vessel support is preferably provided with the at least one position marker in the central area.

It is also preferable if the vessel support and/or the balloon is provided with a coating containing a medicament or is provided directly with an active substance, for which purpose the vessel support can be equipped with a porous surface.

Thus, in this manner known per se, a medicament or active substance that counteracts restenosis, or some other medicament or the respective active substance, can be delivered to the vessel walls over a short or long period of time.

Finally, the present invention relates to a catheter set comprising at least one catheter with the main vessel support and at least one catheter with the novel secondary vessel support.

The advantage of this is that two vessel supports matching each other and adapted to each other are present as a set and therefore do not have to be specially assembled in each case.

Further advantages and features will become clear from the following description and from the attached figures.

It will be understood that the features which are mentioned above and those, which are still to be explained below can be used not only in the combination which is in each case specified but also in other combinations or on their own without departing from the scope of the present invention.

Embodiments of the invention are explained in more detail in the following description in which reference is made to the figures, in which:
- Fig. 1: shows a schematic view of a vessel ramification;
- Fig. 2: shows the vessel ramification from Fig. 1 into which a main stent and a secondary stent have been inserted according to the prior art;
- Fig. 3: shows the vessel ramification from Fig. 1 in which the novel vessel support has been inserted into and positioned in the secondary vessel but has not yet been fully expanded, said vessel support only being de- picted schematically;
- Fig. 4: shows a view similar to Fig. 3, but with the novel vessel support com- pletely expanded;
- Fig. 5: shows a view similar to Fig. 4, in which a vessel support has been inserted into the main vessel but has not yet been completely ex- panded;
- Fig. 6: shows a view similar to Fig. 5, in which the vessel support inserted into the main vessel has been completely expanded;
- Fig. 7: shows a schematic view of a flat projection of the vessel support for the secondary vessel, in which the proximal segment has a closed ring structure and, in the axial direction, has a greater length than the seg- ments of the main section;
- Fig. 8: shows a schematic view of a flat projection of the vessel support for the main vessel;
- Fig. 9: shows a schematic view of the expansion of the vessel support for the main vessel, in which view, for reasons of clarity, the vessel support for the secondary vessel is not shown; and
- Fig. 10: shows a view similar to Fig. 6, in which a schematic representation of the conditions of the vessel supports from Figures 7 and 8 is shown;

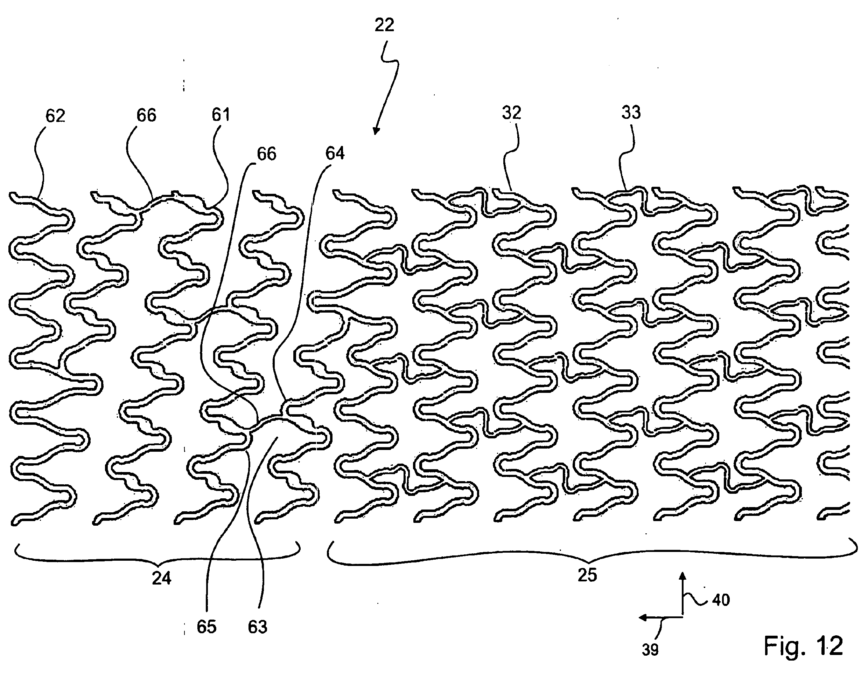

- Fig. 11 and 12: show additional examples that do not form part of the invention;
- Fig. 13: shows a longitudinal sectional view through a balloon of a balloon catheter provided for the vessel support from Figure 7, 11 or 12, in the expanded state;
- Fig. 14: shows, in a view similar to Fig. 8, an alternative structure for the vessel support for the main vessel, where an approximately central area is de- signed as a branch extending in a spiral-shape;
- Fig. 15: shows a view similar to Fig. 14, in which the branch extending in a spiral-shape has axially extending struts; and
- Fig. 16: shows a view similar to Fig. 15, in which the branch extending in a spiral-shape has a connection gap.

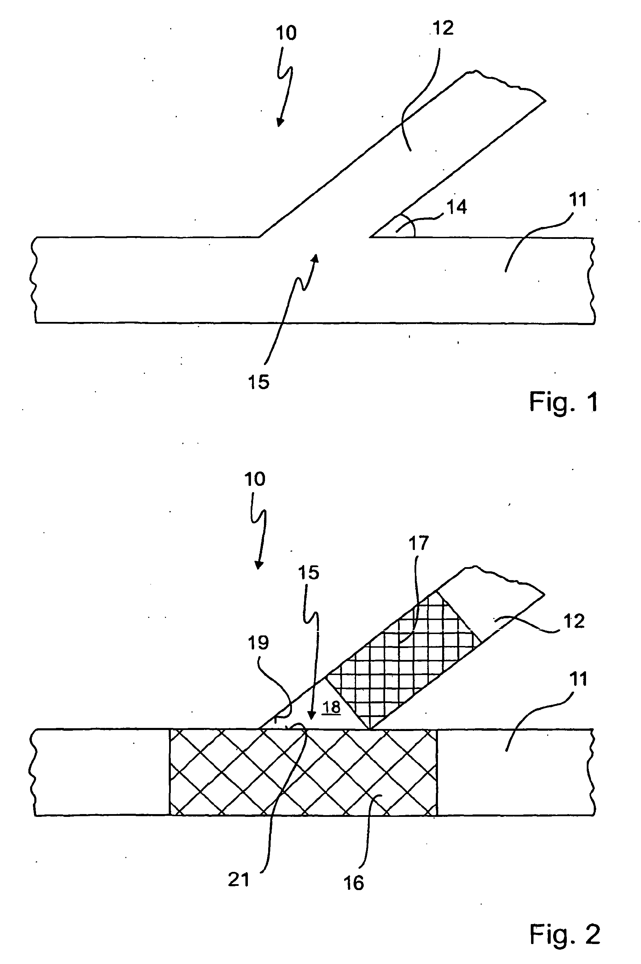

Fig. 1 shows in a schematic view a vessel bifurcation 10 between a main vessel 11 and a secondary vessel 12. The secondary vessel 12 branches off at an angle 14 from the main vessel 11, into which it opens at a vessel opening 15.

It should be noted here that the terms vessel bifurcation, secondary vessel and main vessel are chosen only by way of example and are intended to make the description clearer. Instead of a smaller secondary vessel branching off from a larger main vessel, a vessel ramification may also be present in which a main vessel or secondary vessel divides up into two possibly smaller vessels. The novel vessel supports explained below can be used in any type of such bifurcations.

Fig. 2 shows schematically the situation that arises when known stents are inserted into the vessels 11, 12 and have a purely cylindrical shape in the expanded state. A main stent 16 sits in the main vessel 11, and a secondary stent 17 sits in the secondary vessel 12. Main stent 16 and secondary stent 17 each have a radially permeable and resilient structure, as is indicated by cross-hatching.

There are now essentially two problems that arise:
First, the secondary stent 17 does not cover an area 18 of the wall 19 of the secondary vessel 12 lying opposite the vessel opening 15, with the result that it is not possible to provide any mechanical support there or to supply medicaments with which the secondary stent 17 is coated.
Second, the main stent 16 lies with a section 21 of its structure opposite the vessel opening 15, with the result that the latter is at least partially covered by material of the main stent 16, which can lead to stenoses.

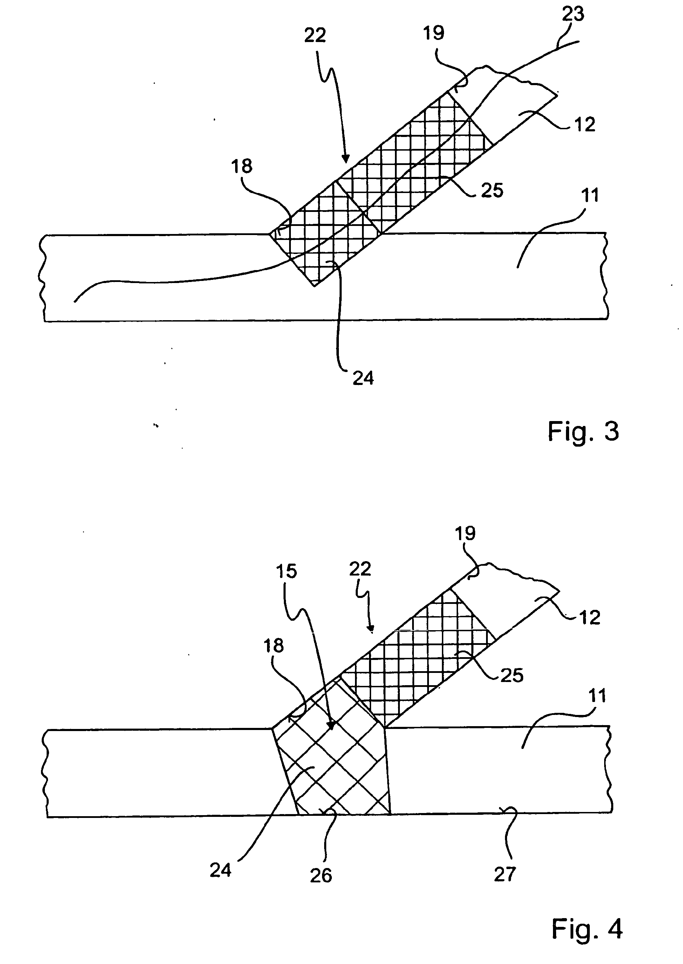

Fig. 3 shows how a novel secondary vessel support 22 is inserted into the secondary vessel 12. Further, the secondary vessel support 22 is positioned such that the area 18 of the wall 19 of the secondary vessel 12 is now covered. The secondary vessel support 22, crimped onto a balloon catheter not shown in Fig. 3, has for this purpose been inserted by way of a guide wire 23 into the secondary vessel 12 to such an extent that its proximal segment 24 protrudes partially into the main vessel 11, while its distal main section 25 lies completely in the secondary vessel 12.

This proximal segment 24 is now expanded by a balloon of the balloon catheter, which balloon is still to be described below, until it assumes a greater radial expansion than the main section 25 and extends into the main vessel 11 such that it comes to lie in the vessel opening 15.

This state is shown in Fig. 4, in which it can be seen that the proximal segment 24 also bears on the wall 27 of the main vessel 11 in an area 26 lying opposite the vessel opening 15. Thereby, the proximal segment 24 is further expanded than the main section 25, as is indicated in Fig. 4 by less dense hatching of the proximal segment 24.

In other words, after expansion, the proximal segment 24 positioned in the vessel opening 15 lies partly in the secondary vessel 12 and partly in the main vessel 11 and supports the walls 19 and 27 there. This mechanical contact not only permits mechanical support of the areas 18 and 26, it also allows a medicament to be delivered there.

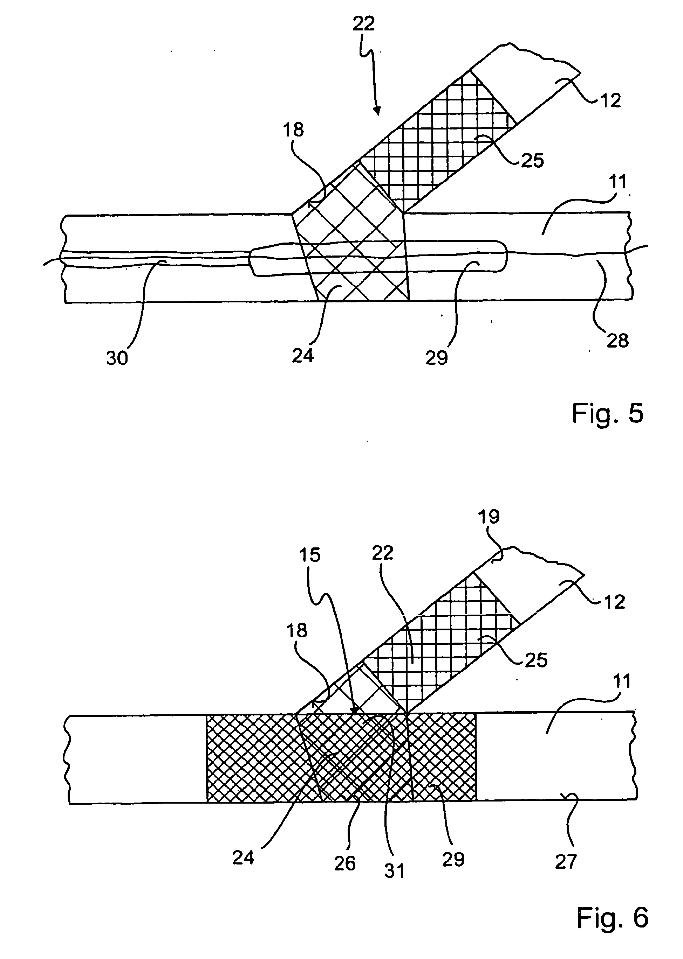

Fig. 5 shows that, by way of a further guide wire 28, an as well novel main vessel support 29 can now be guided through the part of the proximal segment 24 lying in the main vessel 11, said main vessel support 29 sitting on a schematically indicated catheter 30 and being expanded after insertion, as is shown in Fig. 6. In a way still to be described below, the expanded main vessel support 29 has, in the area of the vessel opening 15, a central area 31 that is substantially free of vessel support material, such that the flow, for example of blood, through the vessel opening 15 is not impeded.

By means of the expansion of the proximal segment 24, the latter now has relatively little vessel support material in the area 26 of the wall 27 of the main vessel 11, whereby the novel vessel support 29 also has only a little material in its central area 31 after its expansion. Although the secondary vessel support 22 and the main vessel support 29 therefore intersect in the manner shown, there is only a small overlap of vessel support material in the area 26 of the wall 27 of the main vessel 11. Moreover, the proximal segment 24 also supports the secondary vessel 12 in the area 18 lying opposite the vessel opening.

For the sake of completeness only, it should be noted that, after the expansion of the main vessel support 29, both vessel supports 22, 29 can be dilated again or several times in a manner known per se by means of suitable balloon catheters in order to ensure good positioning and a good mechanical support of both vessels 11, 12 also in the area of the vessel opening 15.

With the novel secondary vessel support 22, one of the two abovementioned problems from the prior art is already avoided in this way, even when a standard stent 16 is used for the main vessel 11, as is shown in Fig. 2. There is no longer an area 18 of the wall 19 of the secondary vessel 12 that is not supported. Nor is there extensive overlapping of both stents, which is known in the prior art and which would increase the rate of restenosis and could lead to thromboses.

Further advantages are afforded, however, when the other novel vessel support described below is used as main vessel support 29, since this additionally ensures that the vessel opening 15 is not covered by vessel support material of the main vessel support 29, thus further reducing the overlapping of the two vessel supports 22, 29.

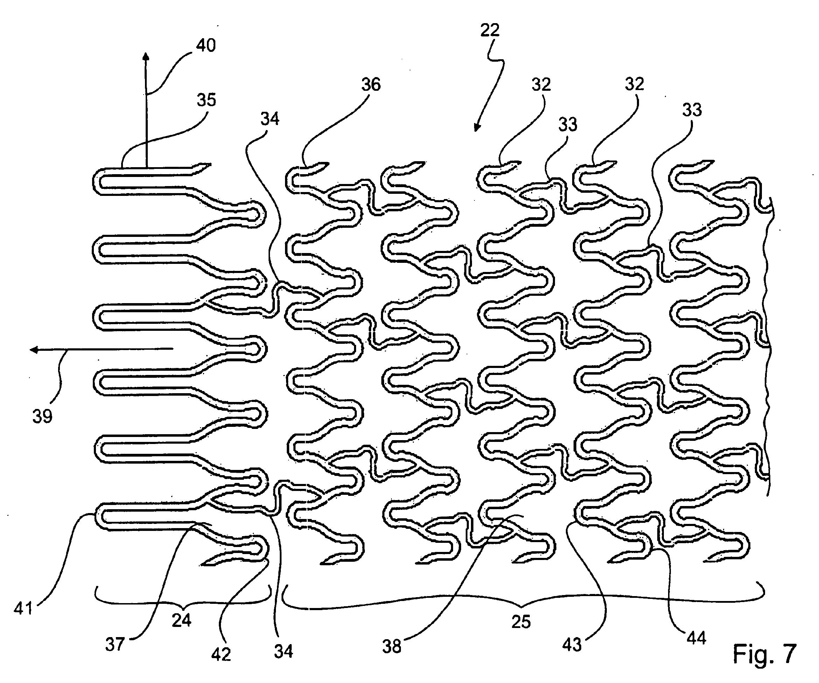

Fig. 7 shows a schematic view of a flat projection of the secondary vessel support 22, the view being cut off at the distal end. The main section 25 is made up of a plurality of segments 32 that are arranged one behind another in the axial direction and that are interconnected via axially extending, undulating struts 33. By way of two further struts 34, the main section 25 directly adjoins the proximal segment 24 which, like the segments 32, has a closed ring structure and is thus able to ensure, after expansion, a good mechanical support of both vessels 11, 12 in the area of the vessel opening 15.

The segments 24, 32 each comprise zigzag-shaped or wave-shaped branches 35, 36, respectively, with loops 37, 38 which lie alongside one another in the circumferential direction 40 and have proximal and distal tips 41, 42 and 43, 44, respectively, in the axial direction 39, wherein the segment 24 in the axial direction 39 has a greater length than the segments 32 of the main section 25. In this way, the segment 24 stores sufficient material in its branch 35 to allow it to be expanded to such an extent that it can be extended into the main vessel 11, as is shown in Fig. 4.

If, in another embodiment not shown here, the extent of the proximal segment 24 in the axial direction 39 is shorter as or the same length as the segments 32 of the main section 25, the number of loops 37 in the branch 35 of the proximal segment 24 is increased, such that a greater number of loops 37 lying alongside one another in the circumferential direction 40 serve as material storage that allow the extending of the proximal segment 24 into the main vessel 11.

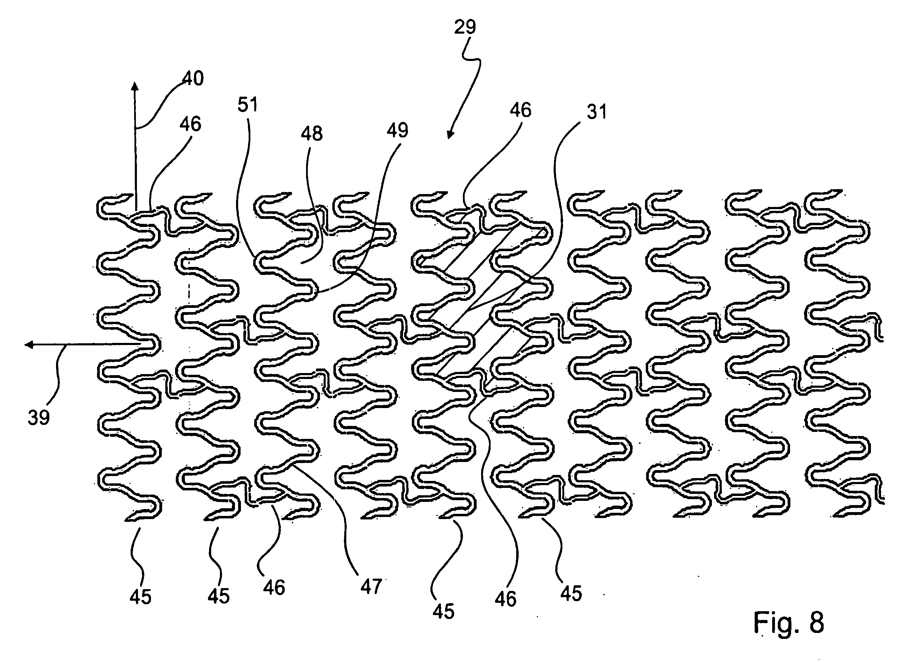

Fig. 8 shows in another schematic view a flat projection of the main vessel support 29, the view being cut off at the distal end. The main vessel support 29 is made up of a plurality of segments 45 that are arranged one behind another in the axial direction 39 and that are each interconnected via two axially extending, undulating struts 46. The segments 45 each comprise a zigzag-shaped or wave-shaped branch 47 with loops 48 that lie alongside one another in the circumferential direction 40 and that have distal and proximal tips 49 and 51 in the axial direction 39.

Between two segments 45 and two associated struts 46, there is then an area, which area is shown by hatching in Fig. 8, that is free of vessel support material and that corresponds to the central area 31 from Fig. 6.

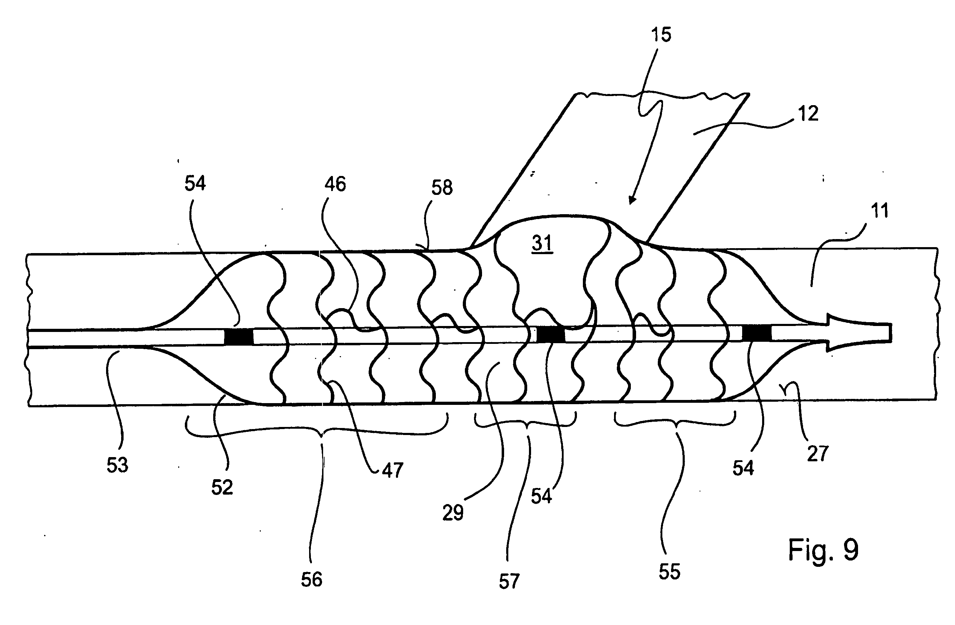

Fig. 9 shows a schematic view of the expansion of the main vessel support 29 placed in the main vessel 11, whereby for reasons of clarity the secondary vessel support 22 is not shown. The main vessel support 29 is arranged on a balloon 52 of a catheter 53 which together form a balloon catheter known per se. The balloon 52 has already been expanded such that it has applied the main vessel support 29 against the wall 27 of the main vessel 11.

Three X-ray markers 54 in total are arranged on the catheter 53 and are used for the exact positioning of the main vessel support 29 in the main vessel 11, such that the central area 31 comes to lie opposite the vessel opening 15. Between its distal section 55 and its proximal section 56, the balloon 52 has a central section 57 where the balloon 52 is approximately sphere-shaped and thus permits a greater expansion than the proximal and distal sections 56 and 55, respectively.

The main vessel support 29 is positioned on the balloon 52 in such a way that its central area 31 lies on the central section 57, with the result that, upon expansion of the balloon 52, the central area 31 opens in the direction to the vessel opening 15.

The balloon 52 is provided here with a coating 58 that contains a medicament, or it can also be coated directly with an active substance. Upon expansion, the active substance or medicament is applied to the wall 27 where it can have, for example, an antiproliferative effect. Alternatively or in addition, the branches 47 and struts 46 can also be provided with such a coating, whereby the branches 47 and struts 46 can also have a porous surface serving as a reservoir for an active substance which, after implantation of the main vessel support 29, is gradually delivered to surrounding tissue. As well the secondary vessel support 22 and the balloon onto which it is crimped can be provided with such a coating, whereby the branches 35, 36 and struts 33, 34 of the secondary vessel support 22 can also have a porous surface that acts as a reservoir for active substance.

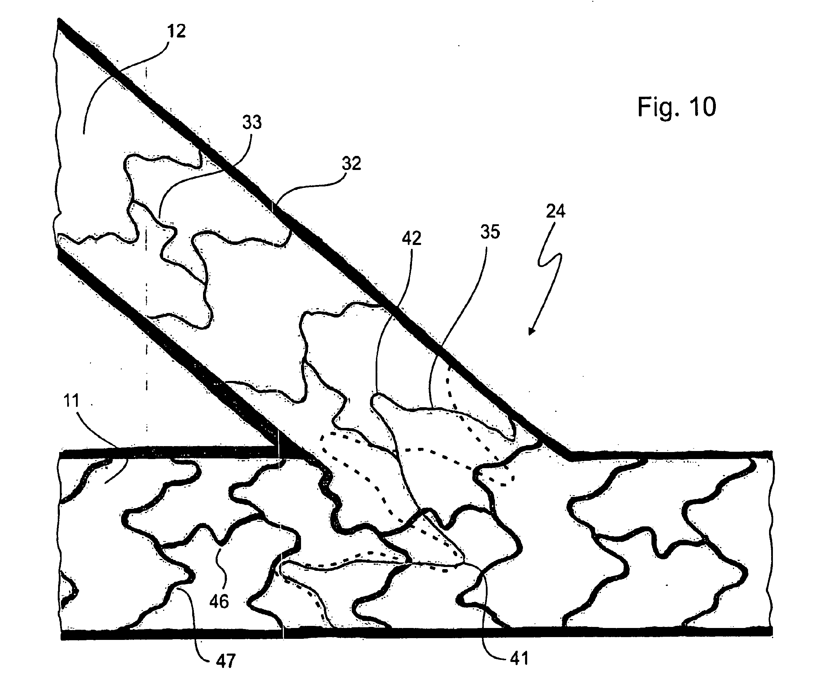

Fig. 10, in a view similar to Fig. 6, but with the vessels 11 and 12 extending in mirror symmetry compared to the arrangement in Figures 1 to 6 and 9, shows a schematic representation of the situation when the vessel supports 22, 29 from Figures 7 and 8 have been inserted into the vessel bifurcation 10, expanded and, if appropriate, further dilated. It can be seen that the proximal segment 24 lies in the vessel opening 15 and bears both on the inside of the secondary vessel 12 and also on the inside of the main vessel 11. The structural elements of the vessel supports 22 and 29 are provided with the same reference numbers as in Figures 7 and 8.

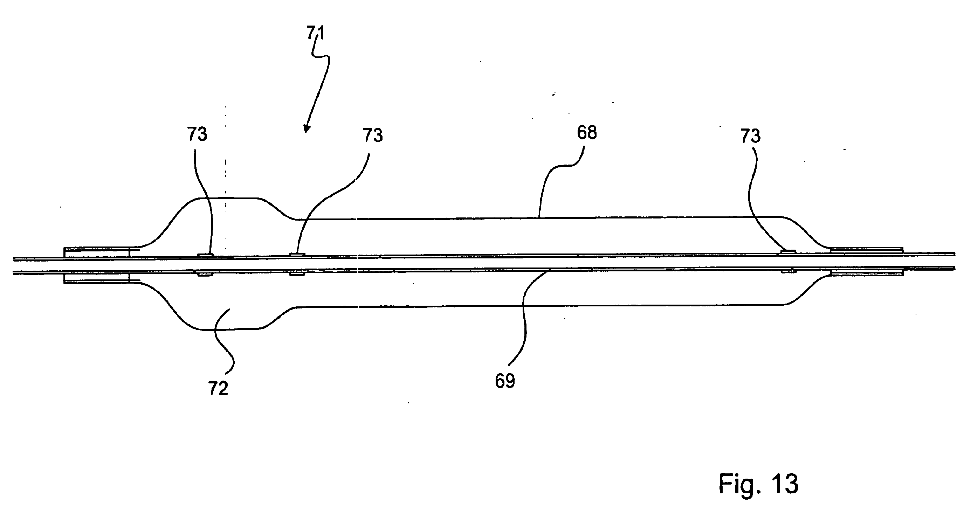

Fig. 13 shows a longitudinal sectional view through a balloon 68 of a balloon catheter provided for the secondary vessel support 22 from Figure 7 the balloon 68 being shown in the expanded state.

The balloon 68 sits in a manner known per se on a catheter 69 through which, during its advance into a vessel, there extends a guide wire not shown in Fig. 13. At its proximal end 71, the balloon 68 has a spherical shape in a section 72 and permits greater expansion there than in its remaining area. The secondary vessel support 22, which is not shown in Fig. 13, is positioned on the balloon 68 such that its proximal segment 24 lies on the section 72, with the result that, upon expansion of the balloon 68, the section 72 extends into the vessel opening 15 and the proximal segment 24 is positioned in the vessel opening in the manner that has already been described above.

Three X-ray markers 73 in total are arranged on the catheter 69 and are used for exact positioning of the secondary vessel support 22 in the secondary vessel 12, such that the proximal segment 24 comes to lie in the vessel opening 15.

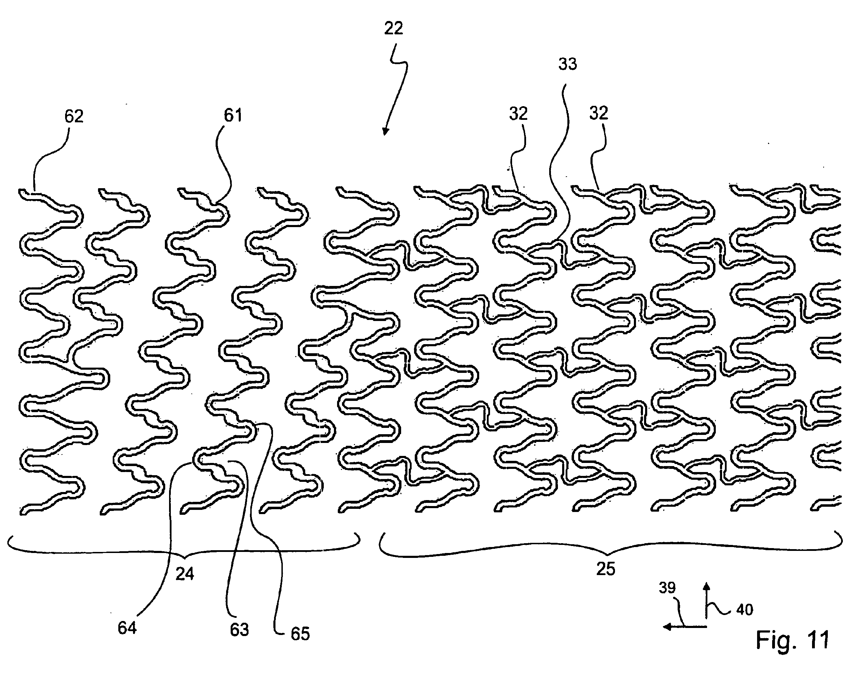

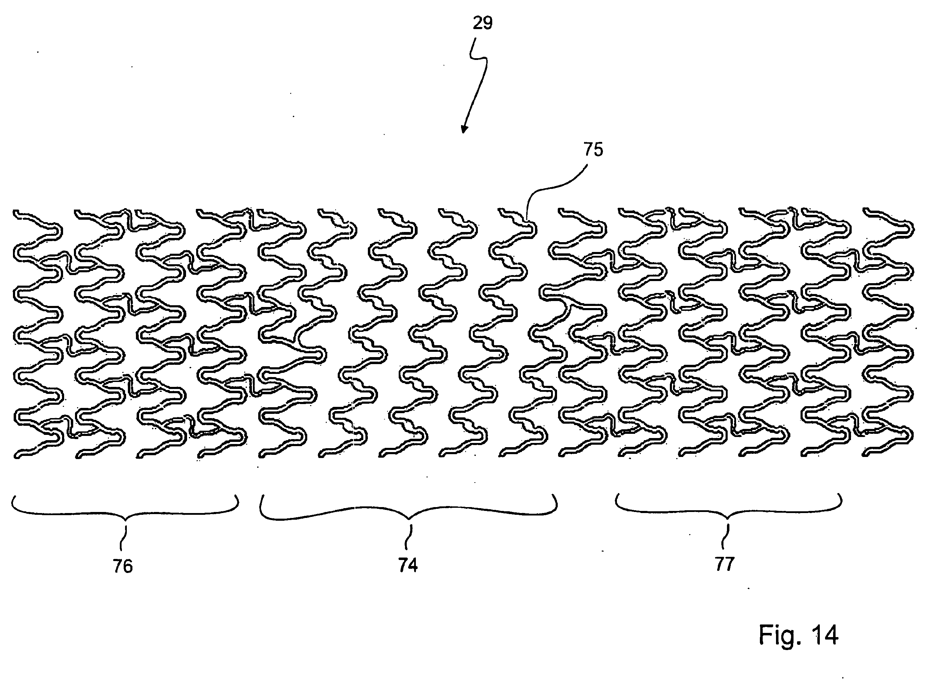

In a view similar to Fig. 8, Fig. 14 shows an alternative structure for the main vessel support 29 in which an approximately central area 74 is designed as a spiral-shaped branch 75, which extends between a proximal and a distal section 76 and 77, respectively. The sections 76 and 77 are designed like the main section 25 described with reference to Fig. 8, while the branch 75 is of a design comparable to the branch 61 from Fig. 11.

The spiral-shaped configuration of the branch 75 has the effect that no positioning problems occur, since the spiral-shaped area 74 can be deformed in the area of the vessel opening 15, largely independently of its axial or circumferential position, such that it completely uncovers the opening. After expansion, the branch 75 thus forms the area 31 free of vessel support material, which has already been discussed above.

It is also advantageous that the spiral-shaped area 74 covers the area 26 of the wall 27 of the main vessel 11 opposite the vessel opening 15 such that spiral-shaped gaps as it were remain, which has the effect that the overlapping with the proximal segment 24 of the secondary vessel support 22 is still further reduced.

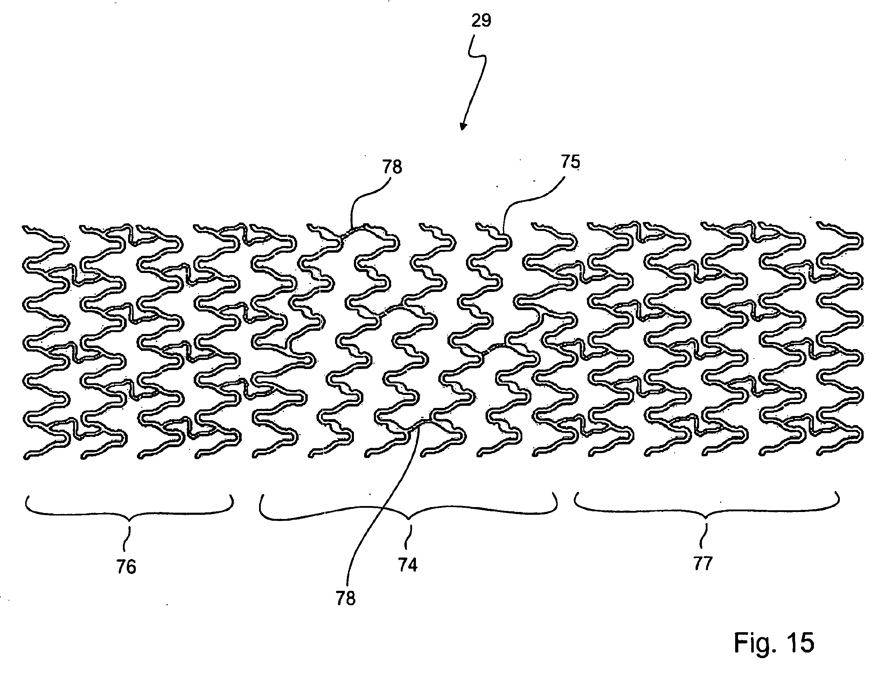

Fig. 15 shows, in a view similar to Fig. 14, a further embodiment of the main vessel support 29 in which the spiral-shaped branch 75 has axially extending struts 78 used for stabilizing purposes.

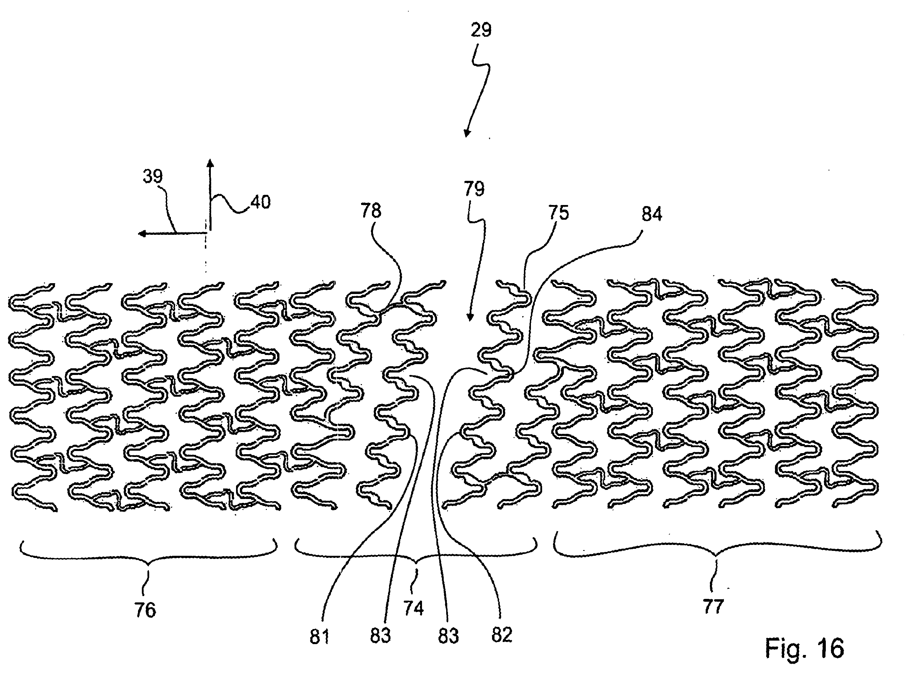

Fig. 16 shows, in a view similar to Fig. 15, a further embodiment of the main vessel support 29 in which the spiral-shaped and also continuous branch 75 has a connection gap 79 which is free of struts 78 and which, after expansion, forms the area 31. The branch 75 again forms tips 81, 82, and also loops 84 that have openings 83.

Compared to the spiral-shaped areas 74 from Figures 14 and 15, the connection gap 79 is distinguished by the fact that a distal tip 81 and a proximal tip 82 lie more or less opposite each other in axial direction 39 and lie at approximately the same height in the circumferential direction 40. In this way, the openings 83 of the loops 84 face each other. This is achieved by the loops 84 being offset in the circumferential direction 40 by about half a width of a loop 84.

## Claims

1. Radially expandable vessel support (22) for implantation in a secondary vessel (12) branching off from a main vessel (11) at a vessel opening (15) and which, in the expanded state, bears on the inside wall (19) of the secondary vessel (12), which has a main section (25) which is to be anchored in the secondary vessel (12) and which is adjoined by a proximal segment (24),
**characterized in that** the proximal segment (24) permits a greater radial expansion than the main section (25) and comes to lie in the vessel opening (15), whereby the proximal segment (24) is directly connected to a segment (32) in the main section (25) via at least one strut (34), such that in the expanded state the proximal segment (24) extends partly into the main vessel (11) and lies with one part of its circumference on the wall (19) of the secondary vessel (12) and with the rest of its circumference on a wall (27) of the main vessel (11).

2. Vessel support according to Claim 1, **characterized in that** the proximal segment (24) comprises a closed ring structure.

3. Vessel support according to Claim 1 or 2, **characterized in that** the proximal segment (24) comprises zigzag-shaped or wave-shaped branches (35, 61) and/or struts (66).

4. Vessel support according to anyone of Claims 1 to 3, **characterized in that** the main section (25) is made up of a plurality of segments (32) that are arranged one behind another in the axial direction (39) and that are connected to one another via axially extending struts (33).

5. Vessel support according to Claim 4, **characterized in that** the proximal segment (24) comprises loops (37) which, in the axial direction (39), have a greater length than the segments (32) of the main section (25) and/or comprises a greater number of loops than segments of the main section.

6. Vessel support according to anyone of Claims 1 to 5, **characterized in that** it is fixed on a balloon (68) of a balloon catheter (68, 69).

7. Vessel support according to Claim 6, **characterized in that** the balloon (68) at its proximal end (71) permits a greater expansion than does the rest of the balloon and is preferably sphere-shaped there.

8. Vessel support according to anyone of Claims 1 to 7, **characterized in that** it is provided, in the area of the proximal segment (24), with at least one position marker, preferably an X-ray marker (73), the position marker preferably being arranged in the area of the proximal end (71) of a balloon (68) of a balloon catheter (68, 69).

9. Vessel support according to Claim 7 or 8, **characterized in that** at least one position marker, preferably an X-ray marker (73), is provided on the balloon (68), the position marker preferably being assigned to the proximal segment (24).

10. Vessel support according to anyone of Claims 1 to 9, **characterized in that** said vessel support and/or a balloon (68) of a balloon catheter (68, 69) is provided with a coating containing a medicament or is coated directly with an active substance.

11. Vessel support according to anyone of Claims 1 to 10, **characterized in that** it comprises a porous surface.

12. Catheter set comprising at least one catheter (69) with the vessel support (22) according to anyone of Claims 1 to 11 and at least one catheter (53) with a vessel support (29) for implantation in a section of the main vessel (11), whrein the vessel support (29) comprises a central area (31, 74) which lies opposite the vessel opening (15) after expansion and is then substantially free of vessel support material.

13. Catheter set according to claim 12, **characterized in that** at least the central area (31, 74) is designed as a spiral-shaped, circumferentially extending branch (75).

14. Catheter set according to Claim 13, **characterized in that** the spiral-shaped, circumferentially extending branch (75) comprises struts (78) expanding in axial direction.

15. Catheter set according to Claim 12 and 13, **characterized in that** the spiral-shaped, circumferentially extending branch (75) comprises a connection gap (79).

16. Catheter set according to anyone of Claims 12 to 15, **characterized in that** the vessel support (29) is fixed on a balloon (52) of a balloon catheter (52, 53).

17. Catheter set according to Claim 16, **characterized in that** the balloon (52) comprises, between its distal and proximal sections (55, 56), a central section (57) that permits a greater expansion than the proximal and distal sections (56,55).

18. Catheter set according to Claim 17, **characterized in that** the balloon (52) is approximately spherical in the central section (57).

19. Catheter set according to anyone of Claims 12 to 18, **characterized in that**, in the area of the central area (31, 74), the vessel support (29) is provided with at least one position marker, preferably an X-ray marker (54).

20. Catheter set according to Claim 19, **characterized in that** the position marker is provided on a balloon catheter (52, 53).

21. Catheter set according to anyone of Claims 12 to 20, **characterized in that** said vessel support and/or a balloon (52) of a balloon catheter (52, 53) is provided with a coating (58) containing a medicament or is coated directly with an active substance.

22. Catheter set according to anyone of Claims 12 to 21, **characterized in that** the vessel support (29) comprises a porous surface.

## Patentansprüche

1. Radial expandierbare Gefäßstütze (22) zur Implantation in ein an einer Gefäßmündung (15) von einem Hauptgefäß (11) abzweigendes Nebengefäß (12), die sich im expandierten Zustand im Nebengefäß (12) innen an dessen Wandung (19) anlegt, und die einen im Nebengefäß (12) zu verankernden Hauptabschnitt (25) aufweist, an den sich ein proximales Segment (24) anschließt,
**dadurch gekennzeichnet, dass** das proximale Segment (24) eine größere radiale Ausdehnung zulässt als der Hauptabschnitt (25) und in der Gefäßmündung (15) zu liegen kommt, wobei das proximale Segment (24) über zumindest eine Strebe (34) direkt mit dem Hauptabschnitt (25) derart verbunden ist, dass sich das proximale Segment (24) in dem expandierten Zustand teilweise in das Hauptgefäß (11) erstreckt und mit einem Teil seines Umfanges an der Wandung (19) des Nebengefäßes (12) und mit seinem restlichen Umfang an einer Wandung (27) des Hauptgefäßes (11) anliegt.

2. Gefäßstütze nach Anspruch 1, **dadurch gekennzeichnet, dass** das proximale Segment (24) eine in sich geschlossene Ringstruktur aufweist.

3. Gefäßstütze nach Anspruch 1 oder 2, **dadurch gekennzeichnet, dass** das proximale Segment (24) zick-zack-förmig oder wellenförmig verlaufende Äste (35, 61) und/oder Streben (66) aufweist.

4. Gefäßstütze nach einem der Ansprüche 1 bis 3, **dadurch gekennzeichnet, dass** der Hauptabschnitt (25) aus mehreren in axialer Richtung (39) hintereinander angeordneten Segmenten (32) besteht, die über axial verlaufende Streben (33) miteinander verbunden sind.

5. Gefäßstütze nach Anspruch 4, **dadurch gekennzeichnet, dass** das proximale Segment (24) Schlaufen (37) aufweist, die in axialer Richtung (39) eine größere Länge aufweisen als die Segmente (32) des Hauptabschnittes (25), und/oder eine größere Anzahl von Schlaufen aufweist als Segmente des Hauptabschnittes.

6. Gefäßstütze nach einem der Ansprüche 1 bis 5, **dadurch gekennzeichnet, dass** sie auf einem Ballon (68) eines Ballonkatheter (68, 69) fixiert ist.

7. Gefäßstütze nach Anspruch 6, **dadurch gekennzeichnet, dass** der Ballon (68) an seinem proximalen Ende (71) eine größere Aufweitung erlaubt als der restliche Ballon und dort vorzugsweise kugelförmig ausgebildet ist.

8. Gefäßstütze nach einem der Ansprüche 1 bis 7, **dadurch gekennzeichnet, dass** sie im Bereich des proximalem Segments (24) mit zumindest einem Positionsmarker, vorzugsweise einem Röntgenmarker (73) versehen ist, wobei der Positionsmarker vorzugsweise im Bereich des proximalen Endes (71) eines Ballons (68) eines Ballonkatheters (68, 69) angeordnet ist.

9. Gefäßstütze nach Anspruch 7 oder 8, **dadurch gekennzeichnet, dass** an dem Ballon (68) zumindest ein Positionsmarker, vorzugsweise ein Röntgenmarker (73) vorgesehen ist, wobei der Positionsmarker vorzugsweise dem proximalen Segment (24) zugeordnet ist.

10. Gefäßstütze nach einem der Ansprüche 1 bis 9, **dadurch gekennzeichnet, dass** sie und/oder ein Ballon (68) eines Ballonkatheters (68, 69) mit einer ein Medikament enthaltenden Beschichtung versehen oder unmittelbar mit einem Wirkstoff beschichtet ist.

11. Gefäßstütze nach einem der Ansprüche 1 bis 10, **dadurch gekennzeichnet, dass** sie mit einer porösen Oberfläche ausgestattet ist.

12. Katheterset, umfassend zumindest einen Katheter (69) mit der Gefäßstütze (22) nach einem der Ansprüche 1 bis 11 und zumindest einen Katheter (53) mit der Gefäßstütze (29) zur Implantation in einen Abschnitt des Hauptgefäßes (11), wobei die Gefäßstütze (29) einen mittleren Bereich (31, 74)) aufweist, der nach Aufweitung der Gefäßmündung (15) gegenüber liegt und dann im Wesentlichen von Gefäßstützenmaterial frei ist.

13. Katheterset nach Anspruch 12, **dadurch gekennzeichnet, dass** zumindest der mittlere Bereich (31, 74) als spiralförmig umlaufender Ast (75) ausgebildet ist.

14. Katheterset nach Anspruch 13, **dadurch gekennzeichnet, dass** der spiralförmig umlaufende Ast (75) in axialer Richtung verlaufende Streben (78) aufweist.

15. Katheterset nach Anspruch 12 und 13, **dadurch gekennzeichnet, dass** der spiralförmig umlaufender Ast (75) eine Verbindungslücke (79) aufweist.

16. Katheterset nach einem der Ansprüche 12 bis 15, **dadurch gekennzeichnet, dass** die Gefäßstütze (29) auf einem Ballon (52) eines Ballonkatheters (52, 53) fixiert ist.

17. Katheterset nach Anspruch 16, **dadurch gekennzeichnet, dass** der Ballon (52) zwischen seinem distalen und proximalen Abschnitt (55, 56) einen mittleren Abschnitt (57) aufweist, der eine größere Aufweitung erlaubt als der proximale und distale Abschnitt (56, 55).

18. Katheterset nach Anspruch 17, **dadurch gekennzeichnet, dass** der Ballon (52) in dem mittleren Abschnitt (57) etwa kugelförmig ausgebildet ist.

19. Katheterset nach einem der Ansprüche 12 bis 18, **dadurch gekennzeichnet, dass** die Gefäßstütze (29) im Bereich des mittleren Bereichs (31, 74) mit zumindest einem Positionsmarker, vorzugsweise einem Röntgenmarker (54) versehen ist.

20. Katheterset nach Anspruch 19, **dadurch gekennzeichnet, dass** der Positionsmarker an einem Ballonkatheter (52, 53) vorgesehen ist.

21. Katheterset nach einem der Ansprüche 12 bis 20, **dadurch gekennzeichnet, dass** die Gefäßstütze und/oder ein Ballon (52) eines Ballonkatheters (52, 53) mit einer ein Medikament enthaltenden Beschichtung (58) versehen oder unmittelbar mit einem Wirkstoff beschichtet ist.

22. Katheterset nach einem der Ansprüche 14 bis 23, **dadurch gekennzeichnet, dass** die Gefäßstütze (29) mit einer porösen Oberfläche ausgestattet ist.

## Revendications

1. Suppor de vaisseau pouvant se déployer radialement (22) pour l'implantation dans un vaisseau secondaire (12) se ramifiant à partir d'un vaisseau principal (11) au niveau d'une ouverture de vaisseau (15) et qui, à l'état déployé, appuie sur la paroi interne (19) du vaisseau secondaire (12) qui comprend une partie principale (25) qui doit être ancrée dans le vaisseau secondaire (12) et qui est jouxte par un segment proximal (24),
**caractérisé en ce que** le segment proximal (24) permet une expansion radiale supérieure à celle de la partie principale (25) et vient reposer dans l'ouverture du vaisseau (15), par lequelle le segment proximal (24) est raccordé directement à un segment (32) dans la partie principale (25) via au moins une entretoise (34) de sorte qu'à l'état déployé, le segment proximal (24) s'étende partiellement dans le vaisseau principal (11) et repose par une partie de sa circonférence sur la paroi (19) du vaisseau secondaire (12), le reste de sa circonférence reposant sur une paroi (27) du vaisseau principal (11).

2. Support de vaisseau selon la revendication 1, **caractérisé en ce que** le segment proximal (24) comprend une structure annulaire fermée.

3. Support de vaisseau selon la revendication 1 ou 2, **caractérisé en ce que** le segment proximal (24) comprend des branches (35, 61) et/ou des entretoises (66) en zigzag ou ondulées

4. Support de vaisseau selon l'une quelconque des revendications 1 à 3, **caractérisé en ce que** la partie principale (25) est constituée d'une pluralité de segments (32) qui sont disposés les uns derrière les autres dans la direction axiale (39) et qui sont raccordés les uns aux autres via des entretoises (33) s'étendant axialement.

5. Support de vaisseau selon la revendication 4, **caractérisé en ce que** le segment proximal (24) comprend des boucles (37) qui, dans la direction axiale (39) ont une longueur supérieure à celle des segments (32) de la partie principale (25) et/ou comprend un nombre de boucles supérieur à celui des segments de la partie principale.

6. Support de vaisseau selon l'une quelconque des revendications 1 à 5, **caractérisé en ce qu'**il est fixé sur un ballonnet (68) d'un cathéter à ballonnet (68, 69).

7. Support de vaisseau selon la revendication 6, **caractérisé en ce que** le ballonnet (68), au niveau de son extrémité proximale (71), permet un déploiement plus important que le reste du ballonnet et y est alors de préférence de forme sphérique.

8. Support de vaisseau selon l'une quelconque des revendications 1 à 7, **caractérisé en ce qu'**il est doté, dans la zone du segment proximal (24) d'au moins un marqueur de position, de préférence un marqueur à rayons X (73), le marqueur de position étant disposé de préférence dans la zone de l'extrémité proximale (71) d'un ballonnet (68) d'un cathéter à ballonnet (68, 69).

9. Support de vaisseau selon la revendication 7 ou 8, **caractérisé en ce qu'**au moins un marqueur de position, de préférence, un marqueur à rayons X (73) est fourni sur le ballonnet (68), le marqueur de position étant de préférence affecté au segment proximal (24).

10. Support de vaisseau selon l'une quelconque des revendications 1 à 9, **caractérisé en ce que** ledit support de vaisseau et/ou un ballonnet (68) d'un cathéter à ballonnet (68, 69) est doté d'un revêtement contenant un médicament ou est revêtu directement avec une substance active.

11. Support de vaisseau selon l'une quelconque des revendications 1 à 10, **caractérisé en ce qu'**il comprend une surface poreuse.

12. Ensemble de cathéter comprenant au moins un cathéter (69) comportant le support de vaisseau (22) selon l'une quelconque des revendications 1 à 11 et au moins un cathéter (53) comportant un support de vaisseau (29) pour l'implantation dans une partie du vaisseau principal (11), le support de vaisseau (29) comprenant une zone centrale (31, 74) qui repose du côté opposé à l'ouverture du vaisseau (15) après déploiement et est ensuite substantiellement dépourvue du matériau de support de vaisseau.

13. Ensemble de cathéter selon la revendication 12, **caractérisé en ce qu'**au moins la zone centrale (31, 74) est conçue comme une branche (75) s'étendant en forme de spirale au niveau circonférentiel.

14. Ensemble de cathéter selon la revendication 13, **caractérisé en ce que** la branche (75) s'étendant en forme de spirale au niveau circonférentiel comprend des entretoises (78) s'étendant en direction axiale.

15. Ensemble de cathéter selon la revendication 12 et 13, **caractérisé en ce que** la branche (75) en forme de spirale au niveau circonférentiel comprend un espace de raccordement (79).

16. Ensemble de cathéter selon l'une quelconque des revendications 12 à 15, **caractérisé en ce que** le support de vaisseau (29) est fixé sur un ballonnet (52) d'un cathéter à ballonnet (52, 53).

17. Ensemble de cathéter selon la revendication 16, **caractérisé en ce que** le ballonnet (52) comprend, entre ses parties distale et proximale (55, 56), une partie centrale (57) qui permet un déploiement plus important que les parties proximale et distale (56, 55).

18. Ensemble de cathéter selon la revendication 17, **caractérisé en ce que** le ballonnet (52) est à peu près sphérique dans la partie centrale (57).

19. Ensemble de cathéter selon l'une quelconque des revendications 12 à 18, **caractérisé en ce que**, dans la région de la zone centrale (31, 74), le support de vaisseau (29) est doté d'au moins un marqueur de position, de préférence un marqueur à rayons X (54).

20. Ensemble de cathéter selon la revendication 19, **caractérisé en ce que** le marqueur de position est disposé sur un cathéter à ballonnet (52, 53).

21. Ensemble de cathéter selon l'une quelconque des revendications 12 à 20, **caractérisé en ce que** ledit support de vaisseau et/ou un ballonnet (52) d'un cathéter à ballonnet (52, 53) est doté d'un revêtement (58) contenant un médicament ou est revêtu directement d'une substance active.

22. Ensemble de cathéter selon l'une quelconque des revendications 12 à 21, **caractérisé en ce que** le support de vaisseau (29) comprend une surface poreuse.
